# EUROPEAN PATENT SPECIFICATION

(11) **EP 2 881 041 B1**
(45) Date of publication and mention of the grant of the patent: **11.01.2017**
(21) Application number: 14192060.3
(22) Date of filing: 06.11.2014
(51) Int. Cl.: A61B 8/00, A61B 8/08, G01S 7/52, G01S 15/89

(54) **Apparatus and method for ultrasonic diagnosis**
Vorrichtung und Verfahren zur Ultraschalldiagnose
Appareil et procédé pour le diagnostic par ultrasons

(30) Priority: 08.11.2013 JP 2013232539
(43) Date of publication of application: 10.06.2015
(73) Proprietor: Hitachi, Ltd., Chiyoda-ku, Tokyo 100-8280 (JP)
(72) Inventor: Tabaru, Marie, Tokyo, 100-8280 (JP); Yoshikawa, Hideki, Tokyo, 100-8280 (JP); Tanaka, Hiroki, Tokyo, 100-8280 (JP)
(74) Representative: MERH-IP Matias Erny Reichl Hoffmann Patentanwälte PartG mbB

(56) References cited:
- WO-A1-2012/116364
- US-A1- 2013 289 402
- Tim Nordenfur: "Comparison of Pushing Sequences for Shear Wave Elastography", Master Thesis, 2 January 2013 (2013-01-02), pages 1-71, XP055186618, Retrieved from the Internet: URL:http://www.diva-portal.org/smash/get/d iva2:633046/FULLTEXT01.pdf [retrieved on 2015-04-29]
- EMILIE MACE ET AL: "In Vivo Mapping of Brain Elasticity in Small Animals Using Shear Wave Imaging", IEEE TRANSACTIONS ON MEDICAL IMAGING, IEEE SERVICE CENTER, PISCATAWAY, NJ, US, vol. 30, no. 3, 2 March 2011 (2011-03-02), pages 550-558, XP011349049, ISSN: 0278-0062, DOI: 10.1109/TMI.2010.2079940

## Description

### BACKGROUND OF THE INVENTION

The present disclosure relates to a technique that measures the viscoelasticity of an examinee by transmitting and receiving ultrasonic waves.

There is a method (an elastography technique) for diagnosing the stiffness of the interior of an examinee from ultrasonic echo signals instead of palpation by a doctor as a diagnostic method for breast cancer, hepatic cirrhosis, angiopathy, and so on. In the diagnosis of stiffness according to the elastography technique, a medical practitioner holds an ultrasound probe against the body surface of a living body, which is an examinee, and applies pressure to cause displacement to tissue in the interior of the examinee. A displacement in the compression direction is estimated from echo signals before and after compressing the biological tissue by applying pressure, and a distortion is determined, which is the spatial differential quantity of the displacement. Moreover, a value related to stiffness, which is the Young's modulus, for example, is calculated from distortion and stress.
This method has a problem in that a subject for imaging is limited to organs in the areas to which pressure is easily applied from the body surface. For example, since a slip surface exists as an intermediate layer between a body surface and a liver, it is difficult to apply pressure to cause a sufficient displacement.

Therefore, there is a technique for diagnosing stiffness in which an acoustic radiation pressure is applied to the interior of an examinee using a displacement generating ultrasonic wave focus beam, that is, a pushing beam or a push pulse, and subject tissue is displaced while suppressing the influence of the intermediate layer.

In this technique, the amount of displacement of tissue taken place in the traveling direction of the focus beam is imaged, or the modulus of elasticity such as a shear modules and a Young's modulus is calculated from the estimation of the propagation velocity of a shear wave taken place in the direction perpendicular to the traveling direction of the focus beam in association with the displacement of the tissue at a focal point. With the use of the technique, an effect is expected that the influence of the intermediate layer caused by the slip surface is reduced, for example.

Moreover, in these years, in an increasing spread of the use of the elastography technique on clinical sites as diagnostic information about chronic hepatitis or tumor, technological development is increasing to implement viscoelasticity measurement including viscosity. Since biological tissue is originally a viscoelastic body having characteristics both of viscosity and elasticity, a highly accurate evaluation of viscoelasticity closer to the actual conditions is expected.

It is necessary for the evaluation of viscoelasticity to calculate the shear modules and the Young's modulus from the group velocity of shear waves as well as to estimate a viscosity parameter such as the viscosity coefficient. For the evaluation of viscoelasticity, there is a method using a particle velocity waveform as disclosed in US Patent Application Publication No. 2007/0038095 and US Patent Application Publication No. 2011/0063950.

Tim Nordenfur: "Comparison of Pushing Sequences for Shear Wave Elastography", Master Thesis, January 2, 2013, pages 1 to 71, XP055186618A describes that shear wave elastography is a medical imaging modality in which tissue elasticity is estimated by measuring the speed of ultrasound-induced shear waves. This study aimed to implement four shear wave generating pushes and compare their performance according to chosen metrics. The focused push, unfocused push, unfocused comb push and line push were implemented on a Verasonics ultrasound system and tested on a polyvinyl alcohol phantom.

Emilie Macé et al: "In Vivo Mapping of Brain Elasticity in Small Animals Using Shear Wave Imaging", IEEE Transactions on Medical Imaging, IEEE Service Center, Piscataway, New York, US, Vol. 30, No. 3, March 1, 2011, pages550 to 558, XP011349049A describes an in vivo mapping of brain elasticity in small animals using shear wave imaging. Specifically, a combination of radiation force and ultrafast ultrasound imaging is used to both generate and track the propagation of shear wave in the brain whose local speed is directly related to stiffness, characterized by the dynamic shear modulus G*.

### SUMMARY

In the evaluation of viscoelasticity, in the case where subject tissue is a viscoelastic body, the propagation velocity of a shear wave is changed depending on the frequency of the shear wave. However, the evaluation of viscoelasticity using the waveform of the particle velocity described above is advantageous in that the evaluation of viscoelasticity is not easily affected by the influence of frequency components such as body motion other than shear waves.

On the other hand, in US Patent Application Publication No. 2007/0038095, an amplitude modulated pushing beam is applied to subject tissue. In order to generate a shear wave at a frequency of about 100 to 1 kHz, a pushing beam is applied for a period of 15 milliseconds at the maximum. Moreover, in US Patent Application Publication No. 2011/0063950, the application time period of a pushing beam for one time is one millisecond. However, it is necessary to apply a pushing beam for a plurality of times for every ten milliseconds (tone burst).

As described above, since the total application time period of the pushing beam is long in the previously existing methods, there is concern that a local temperature rise is increased in the interior of a living body. In order to suppress such a local temperature rise, it is necessary to evaluate viscosity by applying a pushing beam for a fewer number of times of application and a shorter application time period as within one millisecond for one time, for example.

The influence of viscosity on the shear wave is caused by differences in the phase velocity and the damping factor including frequency dependence. Since the influence is amplified in association with the propagation of the shear wave, the influence is basically suited to viscoelasticity measurement as the propagation distance is longer. In other words, one of the preferred conditions for viscoelasticity measurement is to generate a shear wave of a high amplitude, and this means that it is difficult to combine the condition with a constraint caused by the temperature rise described above.

It is an object of the present disclosure to provide an ultrasonic diagnostic apparatus that can solve the problems, suppress a temperature rise in biological tissue at the minimum, and highly accurately analyze a viscosity parameter, and a method for estimating a viscosity parameter.

The above described object is achieved by the invention according to the independent claims. Further preferred developments are described by the dependent claims.

According to an aspect, an ultrasonic diagnostic apparatus according to claim 1 is disclosed, which corresponds to the second embodiment.

Further, according to an aspect, a method according to claim 12 is disclosed, which corresponds to the second embodiment.

According to the present disclosure, it is possible to highly accurately evaluate a viscosity parameter while suppressing a temperature rise in biological tissue at the minimum.

### BRIEF DESCRIPTION OF THE DRAWINGS

FIG. 1 is a diagram of a system configuration of an ultrasonic diagnostic apparatus according to a first embodiment;
FIG. 2 is a diagram illustrative of generating displacement by an ultrasound probe according to the first embodiment;
FIG. 3 is a diagram illustrative of forming ultrasound beams by the ultrasound probe according to the first embodiment;
FIG. 4 is a diagram illustrative of the sequence of calculating a viscosity parameter according to the first embodiment;
FIG. 5A is a diagram of an exemplary shear wave particle velocity detection process according to the first embodiment;
FIG. 5B is a diagram of another exemplary particle velocity detection process according to the first embodiment;
FIG. 6A is a diagram of time waveforms of the displacement of a shear wave according to the first embodiment;
FIG. 6B is a diagram of time waveforms of the particle velocity of a shear wave according to the first embodiment;
FIG. 7A is a diagram of an example of time waveforms of the particle velocity according to the first embodiment;
FIG. 7B is a diagram of another example of time waveforms of the particle velocity according to the first embodiment;
FIG. 8 is a diagram illustrative of a viscoelasticity model; FIG. 9A is a diagram of the frequency dependence of the propagation velocity of a shear wave;
FIG. 9B is a diagram of an example of waveforms of the particle velocity of a shear wave in the case where viscosity is not observed;
FIG. 9C is a diagram of an example of waveforms of the particle velocity of a shear wave in the case where viscosity is observed;
FIG. 10A is a diagram of time waveforms of the particle velocity according to the first embodiment in the case where viscosity is observed;
FIG. 10B is a diagram illustrative of the space attenuation characteristics of the particle velocity according to the first embodiment in the case where viscosity is observed;
FIG. 11A is a diagram illustrative of a process for estimating a viscosity parameter from the positive and negative peaks of time waveforms of the particle velocity according to the first embodiment;
FIG. 11B is a block diagram of a process for estimating a viscosity parameter from time waveforms of the particle velocity according to the first embodiment;
FIG. 12 is a diagram illustrative of waveforms of the particle velocity and space attenuation characteristics according to the first embodiment;
FIG. 13A is a diagram illustrative of the space attenuation characteristics of a shear wave according to the first embodiment;
FIG. 13B is a diagram illustrative of a process for estimating a viscosity parameter from the space attenuation characteristics of a shear wave according to the first embodiment;
FIG. 14A is a diagram of exemplary display of a viscosity parameter according to the first embodiment;
FIG. 14B is a diagram of exemplary display of a viscosity parameter according to the first embodiment;
FIG. 15 is a diagram of a system configuration of an ultrasonic diagnostic apparatus according to a second embodiment;
FIG. 16 is a diagram of the sequence of calculating a viscosity parameter according to the second embodiment;
FIG. 17A is a diagram illustrative of a ROI resetting process according to the second embodiment;
FIG. 17B is a diagram of exemplary display of the ROI resetting process according to the second embodiment; and
FIG. 18 is a diagram of a determining process for the region of interest according to the second embodiment.

### DETAILED DESCRIPTION

In the following, embodiments of the present disclosure will be described with reference to the drawings. In the drawings, the same numbers show the same components.

### First Embodiment

A first embodiment, which is outside the scope of the claims, is an embodiment of an ultrasonic diagnostic apparatus and a method for estimating a viscosity parameter. The ultrasonic diagnostic apparatus includes a displacement generating unit 10 that applies an acoustic radiation pressure to the interior of an examinee to cause displacement, a displacement detecting unit 30 that transmits and receives an ultrasonic wave at a plurality of positions, at least two or more of positions, on the examinee and detects the particle velocity of a shear wave generated in the interior of the examinee, and a viscoelasticity analyzing unit 34 that estimates a viscosity parameter from the particle velocity, in which the viscoelasticity analyzing unit 34 estimates a viscosity parameter from a temporal extent of a waveform of the particle velocity.

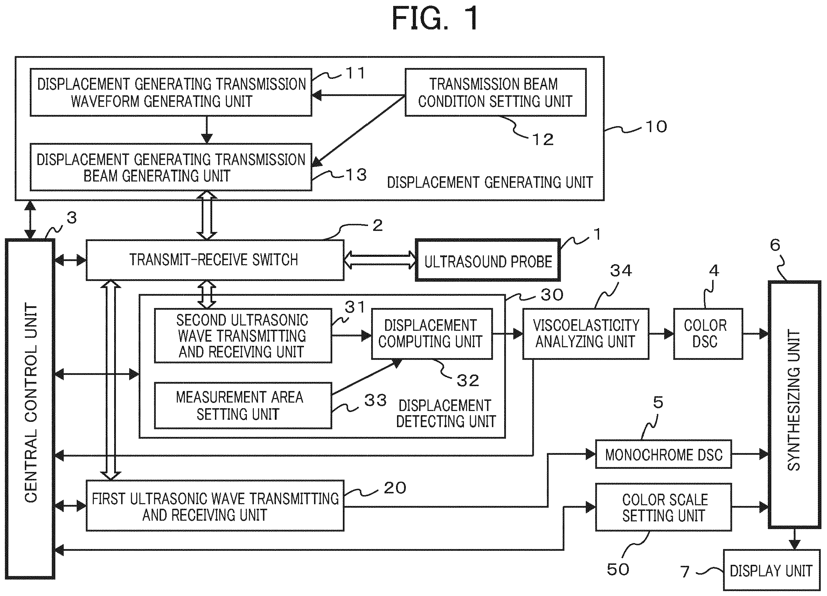

FIG. 1 is an exemplary overall structure of the apparatus according to the first embodiment, which is outside the scope of the claims. An ultrasound probe 1 is used in contact with the skin of an examinee, not illustrated, and includes an ultrasonic wave transmitting and receiving surface on which a plurality of oscillators transmitting and receiving ultrasonic waves with the examinee is arrayed. The components of the apparatus include the following block: a displacement generating unit 10 that causes displacement in the interior of the examinee; a transmit-receive switch 2; a first ultrasonic wave transmitting and receiving unit 20; a displacement detecting unit 30 that detects displacement and includes a second ultrasonic wave transmitting and receiving unit 31; a viscoelasticity analyzing unit 34; and a color scale setting unit 50. These blocks are controlled by a central control unit 3.

The ultrasound probe 1 is connected to a displacement generating transmission beam generating unit 13, the first ultrasonic wave transmitting and receiving unit 20, and the second ultrasonic wave transmitting and receiving unit 31 through the transmit-receive switch 2. The transmit-receive switch 2 is controlled by the central control unit 3 in such a manner that the transmit-receive switch 2 connects and disconnects the ultrasound probe 1 to and from the displacement generating transmission beam generating unit 13, the first ultrasonic wave transmitting and receiving unit 20, and the second ultrasonic wave transmitting and receiving unit 31.

The first ultrasonic wave transmitting and receiving unit 20 is controlled by the central control unit 3 in such a manner that the unit 20 uses a waveform generated at a displacement detecting transmission waveform generating unit, not illustrated, to apply a delay time or a weight to transmission signals of the oscillators of the ultrasound probe 1 and a displacement detecting ultrasonic wave beam, that is, a pushing beam or a push pulse, is focused on a focal point, which is a desired position on the examinee. Echo signals are reflected in the interior of the examinee, returned to the ultrasound probe 1, converted into electrical signals at the ultrasound probe 1, and transmitted to the first ultrasonic wave transmitting and receiving unit 20. The first ultrasonic wave transmitting and receiving unit 20 includes a signal processing circuit that adds a phase to the echo signals for envelope detection, log compression, bandpass filtering, gain control, or the like. Output signals from the first ultrasonic wave transmitting and receiving unit 20 are inputted to a monochrome digital scan converter (DSC) 5. The monochrome DSC 5 can generate a tomogram expressing brightness in monochrome, that is, information of a B mode image.

The displacement generating unit 10 will be described. A transmission beam condition setting unit 12 of the displacement generating unit 10 sets a focusing position, an amplitude value, the number of oscillators of the ultrasound probe 1 used for transmission, a carrier frequency, or the like. A displacement generating transmission waveform generating unit 11 generates a waveform using the carrier frequency set at the transmission beam condition setting unit 12. The displacement generating transmission beam generating unit 13 is controlled by the central control unit 3 in such a manner that the unit 13 uses the waveform generated at the displacement generating transmission waveform generating unit 11 and the setting conditions set at the transmission beam condition setting unit 12, and applies a delay time or a weight to transmission signals of the oscillators of the ultrasound probe 1 to focus the ultrasonic wave beam in the interior of the examinee. Electrical signals from the displacement generating transmission beam generating unit 13 are converted into ultrasound signals at the ultrasound probe 1, and a displacement generating ultrasonic wave beam is applied to the examinee, not illustrated.

The displacement detecting unit 30 will be described. At a measurement area setting unit 33 in the displacement detecting unit 30, the area to measure displacement is set. The second ultrasonic wave transmitting and receiving unit 31 is controlled by the central control unit 3 in such a manner that the unit 31 uses the waveform generated at the displacement detecting transmission waveform generating unit, not illustrated, and the measurement area set at the measurement area setting unit 33, and applies a delay time or a weight to transmission signals of the oscillators of the ultrasound probe 1 to focus the displacement detecting ultrasonic wave beam on a desired position and on the focal point on the examinee, not illustrated. Echo signals are reflected in the interior of the examinee, returned to the ultrasound probe, converted into electrical signals at the ultrasound probe 1, and transmitted to the second ultrasonic wave transmitting and receiving unit 31.

The second ultrasonic wave transmitting and receiving unit 31 of the displacement detecting unit 30 includes a signal processing circuit that adds a phase to the echo signals for envelope detection, log compression, bandpass filtering, gain control, or the like. The output signal from the second ultrasonic wave transmitting and receiving unit 31 is inputted to the displacement computing unit 32. Moreover, the displacement computing unit 32 computes the displacement or the particle velocity of the portions by a correlation operation described later in detail. Furthermore, information about the displacement or the particle velocity outputted from the displacement computing unit 32 is inputted to the viscoelasticity analyzing unit 34.

The viscoelasticity analyzing unit 34 analyzes and estimates the propagation velocity of a shear wave and a viscosity parameter based on the inputted information. The value of the viscosity parameter analyzed and estimated at the viscoelasticity analyzing unit 34 is transmitted to a color DSC 4. The color DSC 4 uses the value of the viscosity parameter to form information about a tomogram expressing viscoelasticity as color information. The output from the color DSC 4 is synthesized at a synthesizing unit 6, and displayed on a screen of a display unit 7 on which a B mode image is also displayed on the screen.

Moreover, the viscoelasticity information such as a viscosity parameter calculated at the viscoelasticity analyzing unit 34 is transmitted to the color scale setting unit 50 through the central control unit 3. The color scale setting unit 50 generates a color scale corresponding to a viscoelasticity color image based on the value of viscoelasticity such as a viscosity parameter. As described later, preferably, the color scale is displayed on the display unit 7 as adjacent to a color image that expresses the magnitude of viscoelasticity and is displayed as superposed, for example, on the B mode image.

It is noted that the central control unit 3, the viscoelasticity analyzing unit 34, and the like, which are a part of the components illustrated by the blocks in FIG. 1, can be implemented by executing a program by a central processing unit (CPU). For the CPU, a typical computer can be used, which includes a CPU, a storage unit (a memory), and an input/output unit. In this case, the display, for example, of the input/output unit of the computer can be used for the display unit 7.

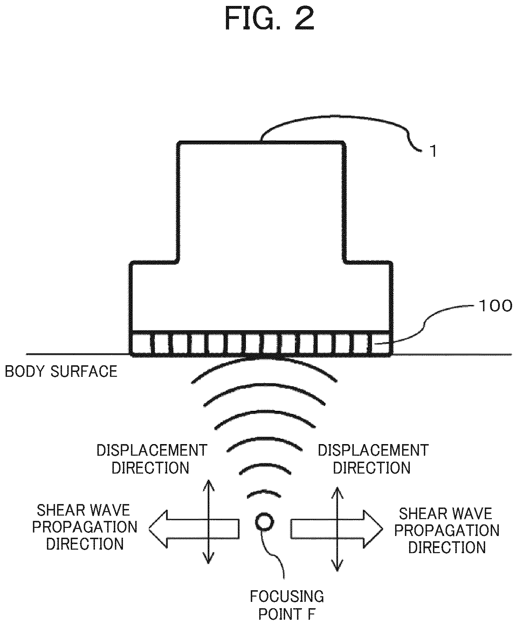

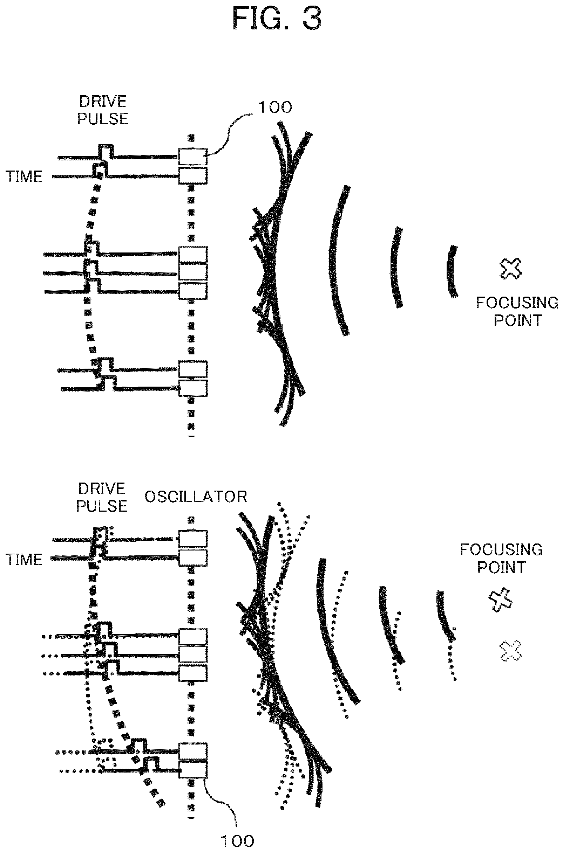

In the embodiment, the case will be described where the linear array ultrasound probe 1 configured of a plurality of oscillators 100 illustrated in FIG. 2 is touched to the body surface of the examinee, and a displacement generating ultrasonic wave beam is focused on the targeted fault plane in the interior of a body. Here, the case will be described where the propagation direction of a displacement generating ultrasonic wave beam is a direction perpendicular to the body surface on a desired fault plane. As illustrated in FIG. 3, an ultrasound beam is formed in which distances between the focal points and the positions of the oscillators 100 of the ultrasound probe 1 are individually determined, the difference in the distance among the oscillators is divided by the sonic speed of the target to calculate a delay time, and the delay time is individually given to the oscillators for transmission. The lower part in FIG. 3 schematically illustrates the case where a focusing point, which is a focal point, is scanned to the upper side as compared with the upper part in FIG. 3.

As illustrated in FIG. 2, a focus beam is applied to the focal point (the focusing point F in FIG. 2), and then a radiation pressure is generated according to the absorption or the scattering of an ultrasonic wave in association with propagation. Generally, the radiation pressure becomes at the maximum at the focal point, and displacement occurs in biological tissue in the focal point region. Moreover, the application of the focus beam is stopped, and then the amount of displacement is relaxed. The generation of the radiation pressure causes a shear wave in the direction in parallel with the surface of the examinee as the focusing point F is a starting point as illustrated in FIG. 2. The direction of displacement is the direction perpendicular to the body surface.

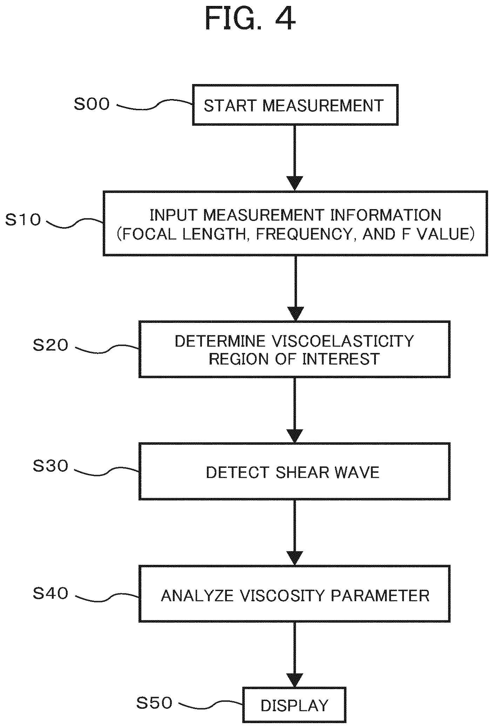

Next, the process flow of viscoelasticity measurement of a viscosity parameter, for example, according to the first embodiment, which is outside the scope of the claims, will be described with reference to FIG. 4. First, in Step 500, viscoelasticity measurement is started. A start signal is inputted using an input device, not illustrated, such as the keyboard or the mouse of a computer. Before starting viscoelasticity measurement, a typical B mode image is displayed on the display unit 7. In Step S10, measurement information about viscoelasticity measurement is inputted. The measurement information includes the depth (i.e. the focusing point F) of the displacement generating ultrasonic wave beam from the body surface, the F value (= the focal length/the aperture diameter), the value of the carrier frequency, and so on. The position of the focal point of the displacement generating ultrasonic wave beam, the F value, the carrier frequency, and the like are inputted to the central control unit 3, and the position of the focal point, the F value, and the carrier frequency are inputted to the transmission beam condition setting unit 12 through the central control unit 3. It may be fine that the measurement information is inputted using the input device, not illustrated, or values suited to the portions such as a mammary gland, a prostate, a blood vessel, and a liver are read out of the storage medium of the storage unit, not illustrated, at the central control unit 3.

In Step S20, the region of interest (ROI) of viscoelasticity is determined. The ROI is inputted using the input device, and inputted to the measurement area setting unit 33 through the central control unit 3. Alternatively, values suited to the portions such as a mammary gland, a prostate, a blood vessel, and a liver are read out of the storage medium at the central control unit 3, and are inputted to the measurement area setting unit 33. For the size of the region of interest ROI, a rectangle is used, which has a width of about 10 mm in the depth direction and a width of about 5 to 20 mm in the direction horizontal to the body surface, for example. The position of the focal point and the ROI are set, and then rasters for use in detection of a displacement (generally, a few µm to a few tens µm) in shear wave propagation and sampling points on the rasters are determined. On the rasters, a pulse repetition frequency (PRF, which is the frequency of a pulse repeatedly transmitted) for receiving a displacement detecting beam is set so as to satisfy the Nyquist's theorem with respect to the expected frequency of the shear wave. For example, in the case where the direction of the raster is the same as the direction of the displacement of a shear wave, the PRF is set to twice the frequency of the shear wave or greater.

In Step S30, the measurement conditions determined in Step S10 are used to apply a displacement generating ultrasonic wave beam from the ultrasound probe 1, and a shear wave is detected in the region of interest determined in Step S20. The displacement of the shear wave or the particle velocity is calculated at the displacement computing unit 32. The displacement computing unit 32 is illustrated as a part of the displacement detecting unit 30. However, the displacement computing unit 32 may be implemented by processing a program at the CPU described above. In Step S40, a viscosity parameter is analyzed at the viscoelasticity analyzing unit 34 using the detected displacement of the shear wave or the particle velocity. In Step S50, the result of analysis and estimation of the viscosity parameter is displayed on the display unit 7, and viscoelasticity measurement in the configuration according to the embodiment is finished.

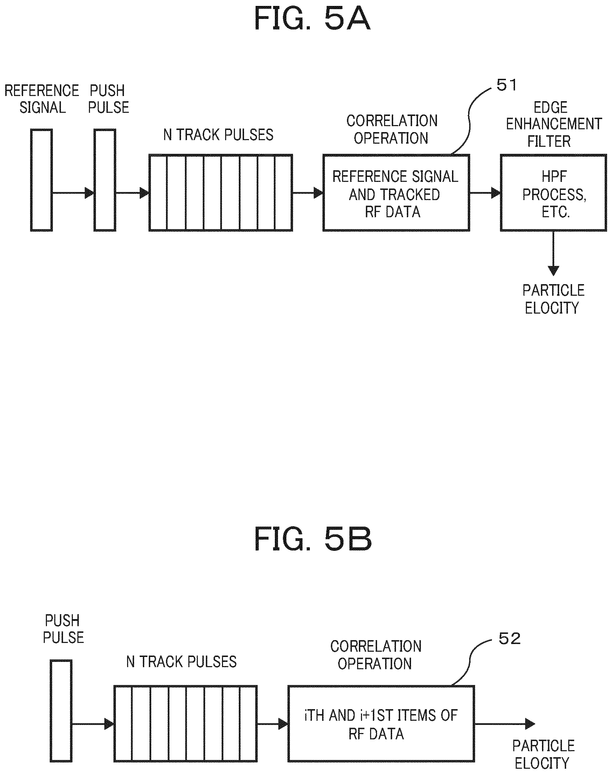

Here, the process flow of detecting a shear wave in Step S30 in FIG. 4 will be described in detail with reference to FIGS. 5A and 5B. Here, the case will be described where the linear array ultrasound probe 1 is touched to the body surface of the examinee, and a displacement generating ultrasonic wave beam, that is, a pushing beam or a push pulse is focused on the targeted fault plane in the interior of a body. Moreover, the case will be described where the propagation direction of a displacement generating ultrasonic wave beam is a direction perpendicular to the body surface on a desired fault plane. In the configuration according to the embodiment, the particle velocity of a shear wave is detected, which the displacement of a shear wave is differentiated with respect to time.

In the case of FIG. 5A, first, a displacement detecting ultrasonic wave beam is applied from the second ultrasonic wave transmitting and receiving unit 31, and a reference signal is acquired. Subsequently, a pushing beam that is a displacement generating ultrasonic wave beam or a push pulse is applied to a focal point. Subsequently, in order to detect a shear wave generated by applying the pushing beam or the push pulse, a displacement detecting ultrasonic wave beam is applied as a track pulse. Here, the repetition frequency at which a displacement detecting ultrasonic wave beam is applied on the rasters is the PRF, and the total of the number of track pulses detected is N pulses (N is a given integer). Subsequently, the reference signal and the tracked RF data are subjected to a correlation operation 51, and the displacement of the shear wave is calculated. In other words, in FIG. 5A, the particle velocity is calculated by the correlation operation between a plurality of signals received at different time instants at the displacement detecting unit 30, for example. The displacement calculated by the correlation operation 51 is subjected to high pass filter (HPF) processing as an edge enhancement filter in the time direction, and the particle velocity is calculated. It is noted that it may be fine that an edge enhancement filter for time differentiation is used to obtain the particle velocity instead of the HPF.

FIG. 5B is another method for calculating the particle velocity. In FIG. 5B, a track pulse, which is a displacement detecting ultrasonic wave beam, is acquired from 1, 2, to N, in the total of N pulses, and the ith and i + 1st items of radio frequency (RF) data (i is an integer from 1 to N - 1) are subjected to a correlation operation 52. In other words, in FIG. 5B, the particle velocity is calculated by the correlation operation between a plurality of signals received at different time instants at the displacement detecting unit 30, for example. By the method in FIG. 5B, the particle velocity can be directly calculated with no use of a filter such as a HPF, so that calculation time can be shortened.

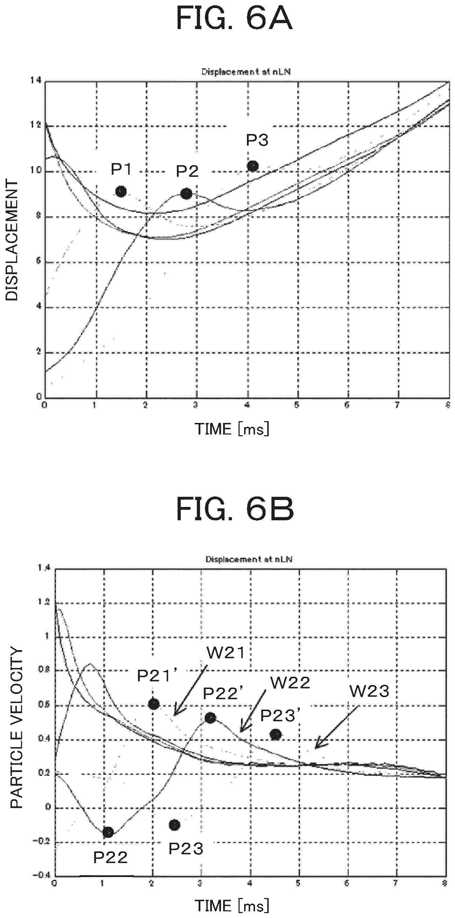

Here, a merit of detecting the particle velocity of a shear wave will be described. FIGS. 6A and 6B are time waveforms in the case where a sine wave of one hertz, for example, which is low frequency noise, is superposed on the displacement of the shear wave or the time waveform of the particle velocity. The low frequency noise means noise that is caused by the movement of the hands or the body motion of a medical practitioner. A plurality of different measurement positions, that is, the peak values of time waveforms of displacement on rasters are illustrated as P1, P2, and P3 in FIG. 6A. Although it is necessary to highly accurately detect peak values for viscoelasticity measurement, it is revealed that it becomes difficult to determine the peak position of displacement as the time instant at which the peak value is found becomes later as P1, P2, and P3.

On the other hand, FIG. 6B illustrates peak values P22, P23, P21', P22', and P23' of time waveforms of the particle velocity. One or two peak values are detected on the time waveform of the particle velocity for the individual rasters. As different from the peaks of the time waveforms of displacement illustrated in FIG. 6A, in the time waveforms of the particle velocity illustrated in FIG. 6B, it is apparent that the peak values can be stably detected even in the case where low frequency noise is superposed, and this is a merit of detecting the particle velocity.

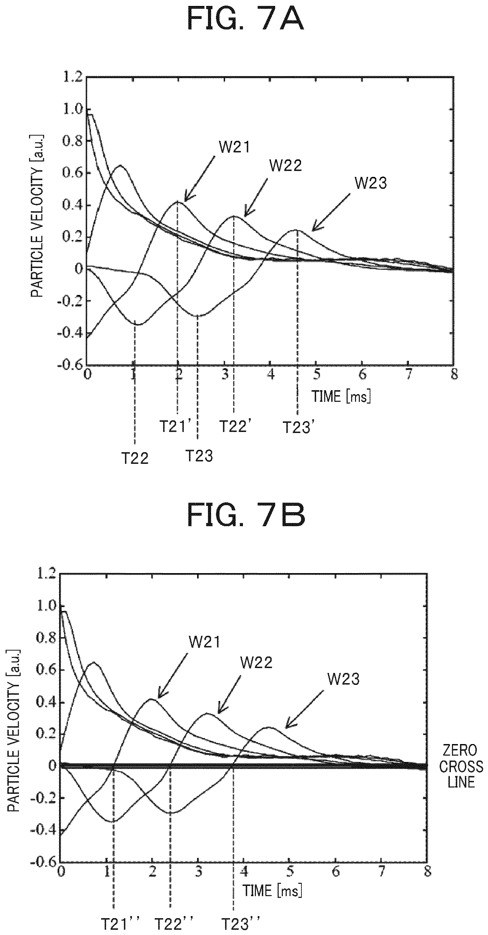

Subsequently, a method for analyzing and estimating a viscosity parameter in Step S40 will be described in detail. FIGS. 7A and 7B are the time waveforms of the particle velocity corresponding to FIG. 6B. Waveforms W21, W22, and W23 of the particle velocity at different measurement positions include one or two time instants at which a peak is taken. Time instants T22 and T23 in FIG. 7A are the time instants of the waveforms W22 and W23 at which a negative peak is taken. Moreover, time instants T21', T22', and T23' in FIG. 7A are the time instants of the waveforms W21, W22, and W23 at which a positive peak is taken. On the other hand, time instants T21", T22", and T23" in FIG. 7B are time instants at which the waveforms W21, W22, and W23 cross zero, that is, a zero cross is taken.

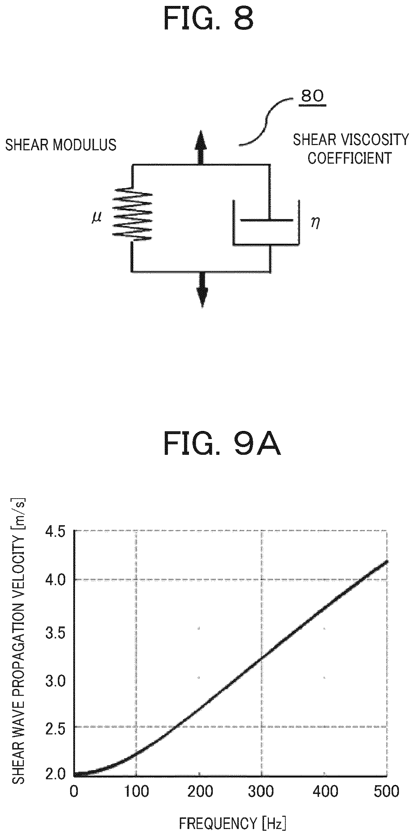

Subsequently, the peak values of the time waveforms of the particle velocity or the time instants at which a peak is taken described above are used to estimate a viscosity parameter. First, the relationship among the viscosity, the peak value, and the time instant at which a peak is taken will be described. FIG. 8 is a Voigt model (a model) 80, which is one of models expressing the viscoelasticity of a living body. The Voigt model 80 is formed of two elements, a shear modulus µ and a shear viscosity coefficient η. It may be fine that a viscoelasticity model of three elements such as a Zener model is considered in addition to the Voigt model 80.

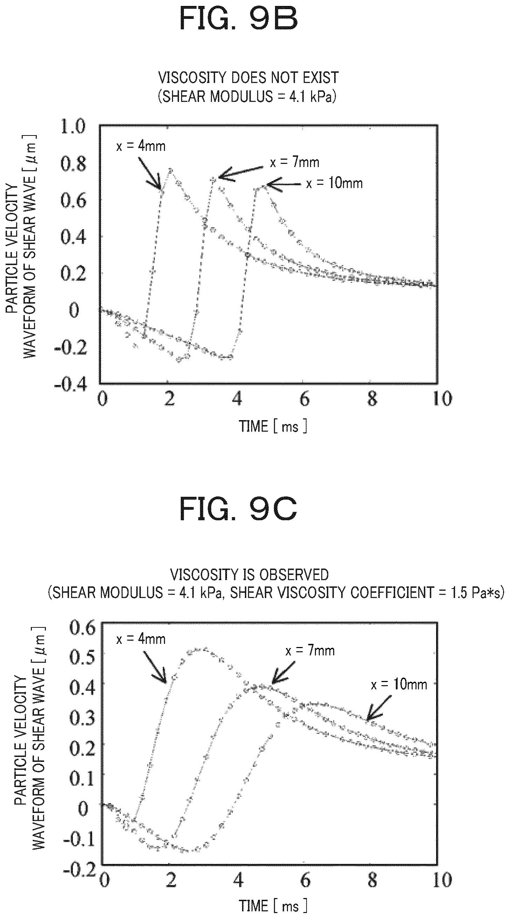

As illustrated in FIG. 9A, when a living body is expressed by the Voigt model illustrated in FIG. 8, the propagation velocity of a shear wave has frequency dispersion characteristics. FIGS. 9B and 9C are examples of waveforms of the particle velocity of a shear wave in the case where the shear viscosity coefficient η is not observed and in the case where the viscosity coefficient η is 1.5 Pa·s. The time waveforms are waveforms at points at a distance x = 4, 7, and 10 mm from a push position, that is, a focal point along the propagation direction of a shear wave. As illustrated in FIG. 9B, in the case where viscosity is not observed, the waveforms of the particle velocity in the time direction are not changed even though the measurement positions are changed from the push position to the distance of 4, 7, and 10 mm. On the other hand, as illustrated in FIG. 9C, in the case where viscosity is observed, time instants at which positive and negative peaks are taken are extended as measurement positions are more apart from the push position. Moreover, it is apparent that the degree of reducing the peak value in association with propagation becomes great when viscosity is observed.

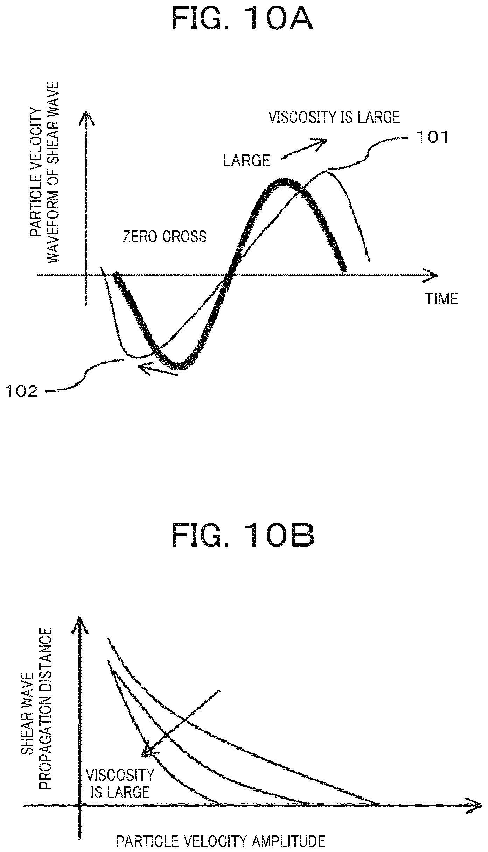

In other words, as schematically illustrated in FIG. 10A, the difference between a time instant at which a positive peak 101 is taken and a time instant at which a negative peak 102 is taken is greater as viscosity is larger. Furthermore, as illustrated in FIG. 10B, it is revealed that the slope of the space attenuation characteristics expressing the amplitude of the peak value and the distance become greater as viscosity is larger.

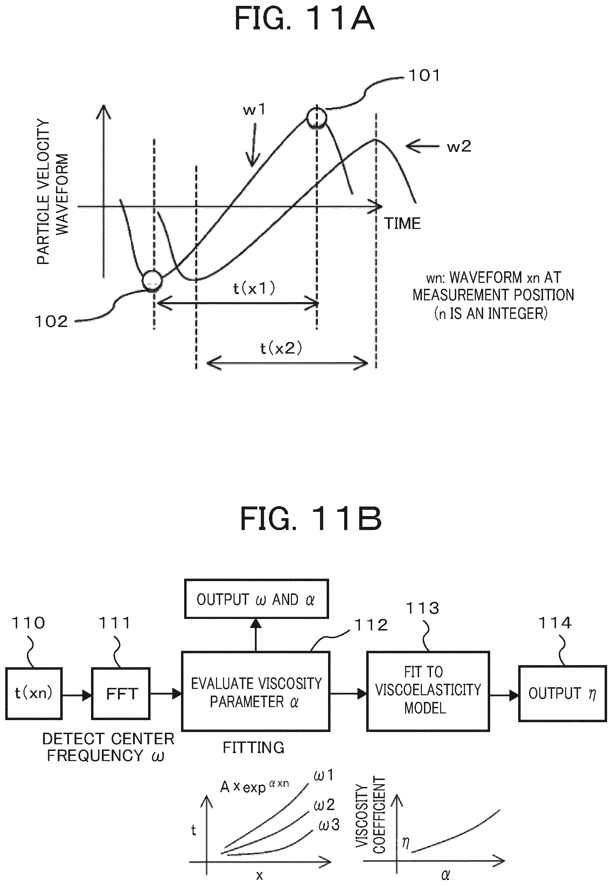

Next, a method for estimating a viscosity parameter according to the embodiment will be described with reference to FIGS. 11A and 11B. FIG. 11A is a time waveform wn of the particle velocity at a measurement position xn and the temporal extent of the waveform of the particle velocity. FIG. 11A is a schematic diagram of an example n = 2, that is, an example that temporal extents of two waveforms of the particle velocity are detected at two positions. In FIG. 11A, suppose that a time difference between a time instant at which a positive peak value is taken and a time instant at which a negative peak value is taken is defined as t(x1) on a time waveform w1 and a time difference is defined as t(x2) on a time waveform w2. The temporal extent of the waveform of the particle velocity can be calculated from the time differences t(x1) and t(x2).

FIG. 11B is a process flow for estimating and analyzing a viscosity parameter using a time difference t(xn) between a positive peak value and a negative peak value, which is an example of the temporal extent of the waveform of the particle velocity of a shear wave. First, at least two or more of time differences t(xn) (n is an integer) 110 are inputted for the temporal extent of the waveform of the particle velocity. Subsequently, a center frequency ω of the waveform of the particle velocity is calculated by an arithmetic operation of FFT 111. In other words, at the viscoelasticity analyzing unit 34, the center frequency of the particle velocity is calculated. The measurement position xn and the time difference t(xn) between a time instant at which a positive peak value is taken and a time instant at which a negative peak value are given by a function unique to the viscoelasticity model with respect to center frequencies ω1, ω2, ω3, and so on. For example, the function is given by A × exp(α·xn) expressing an exponential curve. Therefore, the function is fitted based on information about the detected center frequency ω, the measurement position xn, and the time difference t(xn), and evaluation 112 of a viscosity parameter α is performed. A parameter A and the viscosity parameter α are estimated by fitting in the evaluation 112 of the viscosity parameter α.

In other words, at the viscoelasticity analyzing unit 34, a viscosity parameter is estimated by fitting using an exponential curve, for example, based on the temporal extents of the waveforms of the particle velocity calculated at a plurality of positions.

The center frequency ω and the viscosity parameter α found by fitting, for example, are outputted. The function used here is read on the viscoelasticity analyzing unit 34 from the storage medium, not illustrated, through the central control unit 3. For the function, it may be fine that a known function such as a polynomial function is used, other than the function exp. Moreover, the function is fitted 13 to the viscoelasticity model based on the viscosity parameter α and parameters such as the F value, the focal length, and the carrier frequency, which are the conditions for the ultrasonic wave beam for generating a shear wave, and the viscosity coefficient η of the Voigt model is estimated. Here, it may be fine that the relationship between the viscosity coefficient η and the viscosity parameter α found by fitting are read out of the storage medium of the storage unit, not illustrated, through the central control unit 3 as a look-up table (LUT) or a function. The shear viscosity coefficient η is then outputted 114.

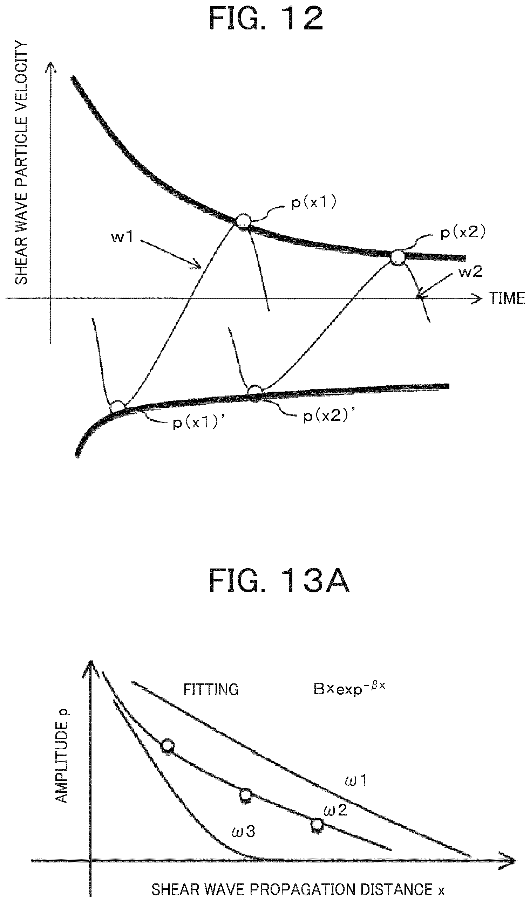

Next, another method for determining a viscosity parameter according to the embodiment will be described. FIG. 12 illustrates measurement positions x1 and x2, positive peak values p(x1) and p(x2), and negative peak values p(x1)' and p(x2)'. In the determining method, the peak value p(xn) or p(xn)' of the particle velocity is used instead of the time difference t(xn) in FIG. 11B. The function B × exp(β·xn) expressing an exponential curve is fitted, and a parameter B or a viscosity parameter β is estimated, instead of the viscosity parameter α or the parameter A.

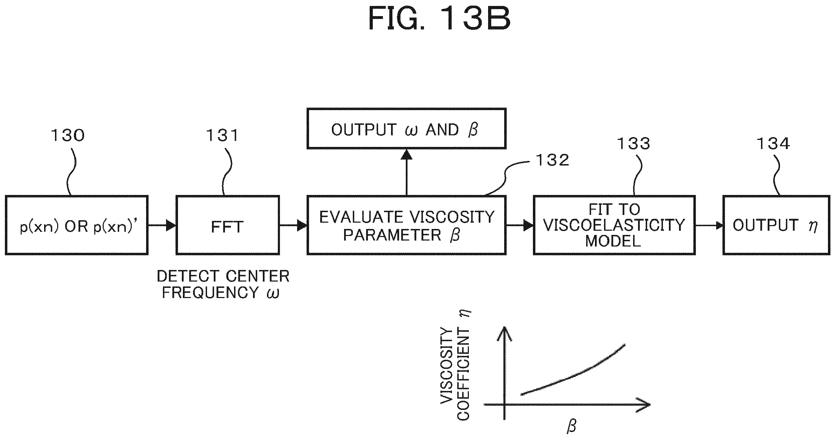

FIGS. 13A and 13B are an example that the peak value p(xn) is inputted 130 for fitting. The center frequency ω is detected by FFT 131, the viscosity parameter is evaluated 132, and then the viscosity parameter and the center frequency ω are outputted as the output values of the viscosity parameter. Moreover, based on the viscosity parameter β and parameters such as the F value, the focal length, and the carrier frequency, which are the conditions for the ultrasonic wave beam for generating a shear wave, the viscosity coefficient η of the Voigt model illustrated in FIG. 8 is estimated by fitting 133. Here, it may be fine that the relationship between the viscosity coefficient η and the viscosity parameter β found by fitting is read out of the storage medium, which is the storage unit, not illustrated, through the central control unit 3 as a LUT or a function. The shear viscosity coefficient η is then outputted 134.

It is noted that for input parameters other than the parameters t(xn), p(xn), and p(xn)' for calculating the temporal extent of the waveform of the particle velocity described above, the following may be used: the integral value of the waveform of the particle velocity; the integral value of the particle velocity before and after a time instant at which a zero cross value is taken; the difference of the integral value of the waveform of the particle velocity between a time instant later and a time instant earlier the time instant at which zero is taken; the integral quantity of the particle velocity greater or smaller than a given threshold; the difference between a time instant at which a positive peak is taken and a time instant at which zero is taken; and the difference between a time instant at which a negative peak is taken and a time instant at which zero is taken, for example. In other words, it is also possible that the temporal extent of the waveform of the particle velocity of a shear wave is calculated from a time instant at which the particle velocity takes a zero cross value or the integral value of the particle velocity before and after a time instant at which a zero cross value is taken, for example, instead of calculating the temporal extent from two time instants, a time instance at which the particle velocity takes a positive value and a time instance at which the particle velocity takes a negative value. Moreover, in FIGS. 9A to 12, the negative peak is detected at a time instant earlier than the positive peak is detected. However, the viscosity parameter can be similarly determined as the positive peak is detected at a time instant earlier than the negative peak is detected.

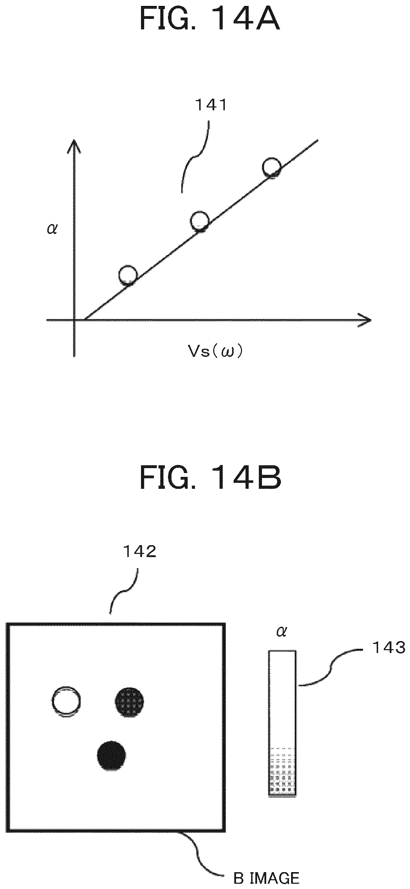

FIGS. 14A and 14B are an example of the viscosity parameter α displayed on the display unit 7 of the ultrasonic diagnostic apparatus according to the embodiment. For example, as illustrated in FIG. 14A, a shear wave propagation velocity Vs (ω) with respect to the viscosity parameter α and the center frequency can be plotted and displayed 141. The propagation velocity Vs (ω) is calculated by a known method at the viscoelasticity analyzing unit 34. In addition to the viscosity parameter α, the viscosity parameter β and the shear viscosity coefficient η can be displayed. Alternatively, it may be fine that as illustrated in FIG. 14B, the viscosity parameter α is superposed on a B image displayed on the display unit 7 and a 2D color map 142 is displayed. At this time, it is effective as described above that color scales 143 for the viscosity parameters α and β and the shear viscosity coefficient η are simultaneously displayed on the display unit 7.

### Second Embodiment

A second embodiment is an embodiment of an apparatus and a method for ultrasonic diagnosis. The apparatus includes a displacement generating unit 10 that applies an acoustic radiation pressure to the interior of an examinee to cause displacement, a displacement detecting unit 30 that transmits and receives an ultrasonic wave at a plurality of positions, at least two or more of positions, on the examinee and detects the particle velocity of a shear wave generated in the interior of the examinee, a viscoelasticity analyzing unit 34 that estimates a viscosity parameter from the particle velocity, and a feedback parameter determining unit 38, in which the viscoelasticity analyzing unit 34 estimates a viscosity parameter from the temporal extents of waveforms of the particle velocity, and the feedback parameter determining unit 38 adjusts at least one of a transmission condition and a region of interest in a second measurement, which is the subsequent measurement, based on the viscosity parameter estimated from the temporal extents of the waveforms of the particle velocity obtained in the first measurement.

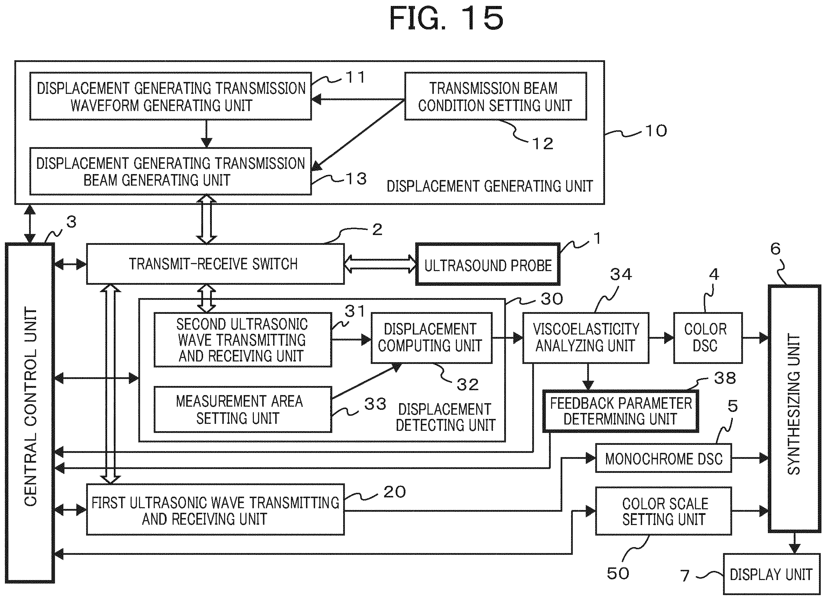

In the following, an ultrasonic diagnostic apparatus according to the second embodiment will be described in which feedback control is performed using the feedback parameter determining unit 38. FIG. 15 is an exemplary overall structure of the ultrasonic diagnostic apparatus according to the second embodiment, and the feedback parameter determining unit 38 is newly added to the ultrasonic diagnostic apparatus in the configuration illustrated in FIG. 1. As similar to the viscoelasticity analyzing unit 34, the feedback parameter determining unit 38 can be implemented by executing a program by a CPU. The blocks of the other components are the same as the blocks in the configuration of the ultrasonic diagnostic apparatus according to the first embodiment, which is outside the scope of the claims, illustrated in FIG. 1, and the overlapping description will be omitted.

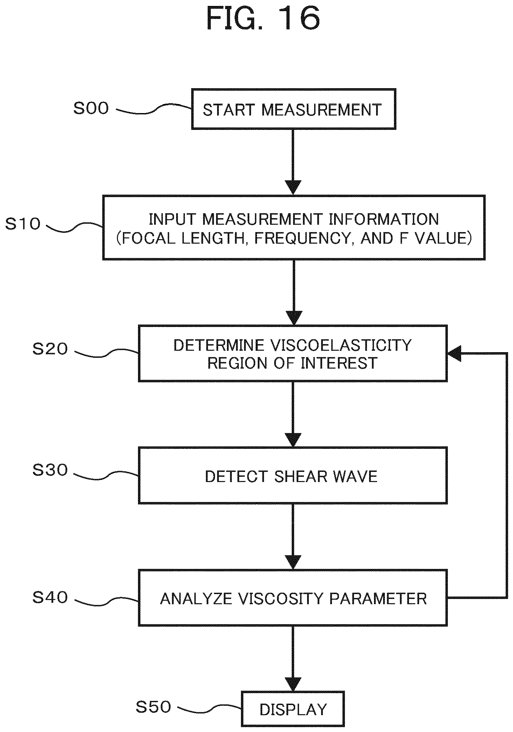

FIG. 16 is the sequence of calculating a viscosity parameter in the ultrasonic diagnostic apparatus according to the second embodiment. In the second embodiment, the value of the viscosity parameter analyzed and estimated in Step S40 is fed back to the determination of the region of interest of the shear wave in Step S20. The value to be fed back is calculated at the feedback parameter determining unit 38. The calculated value is inputted to a measurement area setting unit 33 of the displacement detecting unit 30 through the central control unit 3.

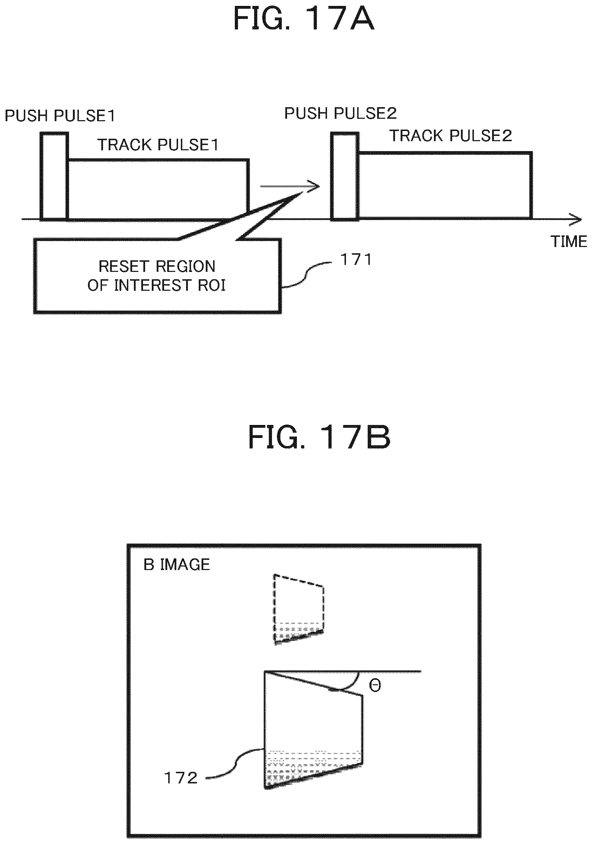

FIG. 17A is the outline of the process flow of viscoelasticity measurement including feedback control according to the embodiment. For example, as described in FIG. 5B, a push pulse 1 and a track pulse 1 are applied, the particle velocity of a shear wave is detected, a viscosity parameter is estimated, and the region of interest ROI is reset 171 based on the estimated value of the viscosity parameter. Since the amplitude value of the shear wave in association with propagation becomes smaller as viscosity is larger, a measurable region becomes smaller. Therefore, the region of interest ROI is reset in such a manner that the size of the region of interest ROI becomes smaller as the value of viscosity is greater. Subsequently, a push pulse 2 and a track pulse 2 are applied to again measure viscoelasticity in the reset region of interest ROI.

As illustrated in FIG. 17B, the size of a region of interest 172 to be superposed on a B image is set using an angle Θ of a trapezoid, for example. In other words, in the embodiment, a preferred example of the shape of the region of interest ROI is a trapezoid, and the value of the angle Θ is more increased as viscosity is larger. In addition to the angle Θ of a trapezoid, the width in the lateral direction and the width in the depth direction of the region of interest ROI may be used. These values are set with respect to the focal point F. In other words, the feedback parameter determining unit 38 changes the size of the region of interest ROI to be adjusted as matched with the measurement position. It is noted that the reset value of the region of interest ROI can be read out of the storage unit, not illustrated, through the central control unit 3 based on the estimated viscosity parameter.

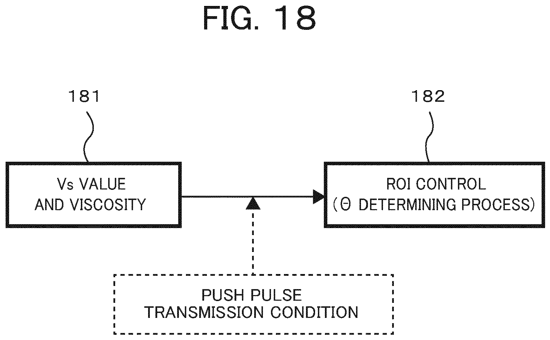

FIG. 18 is a schematic process for determining the region of interest ROI in the feedback parameter determining unit 38 according to the embodiment. Parameters 181 such as the Vs value, the center frequency, the viscosity parameters α and β, and the viscosity coefficient η are used for ROI control 182, and the angle Θ is determined. It may be fine that in determining the angle Θ, the angle Θ is determined further using the conditions for applying the displacement generating ultrasonic wave beam such as the F value, the carrier the frequency, and the focal length, that is, the push pulse transmission conditions and the pushing beam application conditions expressed by a dotted line. In this case, as illustrated in FIG. 17B, the shape of the region of interest ROI is changed depending on the measured depth of the B image. Therefore, the shape of the region of interest ROI is displayed on the display unit 7, and the medical practitioner can visually confirm the region of interest ROI to be determined.

According to the embodiment, it is possible to highly accurately estimate and measure a viscosity parameter by performing feedback control. The shape of the region of interest ROI described above may be a given geometrical shape in addition to a trapezoid. Moreover, the value to be fed back may be the amplitude value, the F value, the carrier frequency, and the like, which are the application conditions for the push pulse, in addition to the region of interest ROI. These values are fed back to allow selection of viscoelasticity measurement such as viscoelasticity measurement in which importance is placed on safety as the amplitude value and the region of interest ROI are reduced, and viscoelasticity measurement in which importance is placed on the region of interest as the amplitude value and the region of interest ROI are increased.

Furthermore, it may be fine that the medical practitioner turns ON/OFF feedback control according to the second embodiment through the input device, not illustrated. In addition, such a configuration may be provided in which a switch that turns ON/OFF feedback control by the feedback parameter determining unit 38 is included. Moreover, it may be fine that the medical practitioner selects parameters for feedback control using the input device, not illustrated. Alternatively, it may be fine that the central control unit 3 automatically sets parameters using information about a measurement target and measured time instants.

In all the embodiments described above, the ultrasonic wave focus beam is used to generate a radiation pressure to cause a shear wave. However, it may be fine that a known method is used in addition to a radiation pressure, including a mechanical drive using a DC motor and a vibration pump, manual pressure, and pressure caused by electric pulses.

Moreover, a convex prove or a sector probe may be used, or a two-dimensional probe may be used instead of the linear array probe. The linear array probe is preferably used for a breast, a tendon, and a muscle, for example. Moreover, the convex prove is preferably used for a liver, and the sector prove is used for a heart. Furthermore, for the oscillators of the ultrasound probe 1, for example, a piezoelectric device made of a ceramic material or a high molecular material or an electrostatic capacitive oscillator made of silicon is used. However, the oscillators are not limited thereto. In the case of using a two-dimensional probe, a shear wave can be three-dimensionally detected, and the propagation of the shear wave can be more clearly displayed. Thus, it is expected as effects that evaluation performance is improved and evaluation time is shortened.

It is noted that in the case where a plurality of spatial elasticities exists in the area to measure viscosity, the viscosity parameter to be measured and the viscosity coefficient are mean values. Therefore, it may be fine that a region having uniform distortion information is selected for the region to detect viscosity as described in International Publication No. WO/2012/105152 filed by the present inventors. In the region having uniform distortion information, the modulus of elasticity is uniform, so that the viscosity parameter and the viscosity coefficient can be highly accurately estimated. The measurement targets include a liver, a chest, a blood vessel, a prostate, a ligament, a tendon, and a muscle, for example.

According to the present disclosure described in detail, the viscosity parameter can be evaluated, so that it is possible to objectively evaluate the tissue characteristics such as tumor or to determine treatment effects. Moreover, an acoustic radiation pressure using a pulse wave is generated, so that a period to apply an acoustic radiation pressure necessary to a single measurement ranges from 0.5 milliseconds to one millisecond, and a temperature rise in biological tissue can be suppressed at the minimum. Furthermore, the waveform of the particle velocity is used, so that measurement robust to body motion, for example, is possible. In addition, it is possible that a region to measure a shear wave is reset based on the viscosity parameter and the viscosity parameter is again evaluated, and that highly accurate measurement is performed.

It is noted that the present disclosure is not limited to the foregoing embodiments, and includes various exemplary modifications. For example, the foregoing embodiments are described in detail for better understanding of the present disclosure, and all the described configurations are not necessarily included. Moreover, a part of the configuration according to an embodiment can be replaced by the configuration according to another embodiment, and the configuration according to an embodiment can be additionally provided with the configuration according to another embodiment. Furthermore, the configurations according to the embodiments can be partially added with, deleted from, and replaced by the other configurations.

In addition, the example is described in which the foregoing configurations, the functionalities, the processing units, and the like are implemented by creating a program that implements a part or all of them. However, it is without saying that it may be fine that a part or all of them are implemented by hardware as by designing a part or all of them using an integrated circuit, for example.

## Claims

1. An ultrasonic diagnostic apparatus comprising:
a displacement generating unit (10) configured to apply an acoustic radiation pressure to an interior of an examinee to cause displacement;
a displacement detecting unit (30) configured to transmit and receive an ultrasonic wave at a plurality of positions of the examinee to detect a particle velocity of a shear wave generated in the interior of the examinee; and
a viscoelasticity analyzing unit (34) configured to estimate a viscosity parameter from the particle velocity,
wherein the viscoelasticity analyzing unit (34) estimates the viscosity parameter from a temporal extent of a waveform of the particle velocity, **characterized by** further comprising a feedback parameter determining unit (38),
wherein based on the viscosity parameter estimated from a temporal extent of a waveform of the particle velocity obtained in a first measurement, the feedback parameter determining unit (38) adjusts at least one of a transmission condition and a region of interest in a second measurement, which is a subsequent measurement.

2. The ultrasonic diagnostic apparatus according to claim 1, wherein the viscoelasticity analyzing unit (34) calculates a temporal extent of a waveform of the particle velocity from two time instants, a time instance at which the particle velocity takes a positive value and a time instance at which the particle velocity takes a negative value.

3. The ultrasonic diagnostic apparatus according to at least one of the claims 1 to 2, wherein the viscoelasticity analyzing unit (34) calculates a temporal extent of a waveform of the particle velocity from an integral value of the particle velocity at a time instant at which the particle velocity takes a zero cross value or before and after a time instant at which the zero cross value is taken.

4. The ultrasonic diagnostic apparatus according to at least one of the claims 1 to 3, wherein the viscoelasticity analyzing unit (34) estimates the viscosity parameter by fitting based on a temporal extent of a waveform of the particle velocity calculated at the plurality of positions.

5. The ultrasonic diagnostic apparatus according to claim 4, wherein the viscoelasticity analyzing unit (34) performs the fitting using an exponential curve.

6. The ultrasonic diagnostic apparatus according to at least one of the claims 1 to 5, wherein the viscoelasticity analyzing unit (34) calculates a center frequency of the particle velocity.

7. The ultrasonic diagnostic apparatus according to at least one of the claims 1 to 6, wherein the particle velocity is calculated by a correlation operation between a single reference signal and a plurality of signals received at the displacement detecting unit (30) at different time instants.

8. The ultrasonic diagnostic apparatus according to at least one of the claims 1 to 7, wherein the particle velocity is calculated by a correlation operation between a plurality of signals received at the displacement detecting unit (30) at different time instants.

9. The ultrasonic diagnostic apparatus according to claim 8, wherein the feedback parameter determining unit (38) changes a size of the region of interest to be adjusted as corresponding to a measurement position.

10. The ultrasonic diagnostic apparatus according to claim 9, wherein a shape of the region of interest is a trapezoid.

11. The ultrasonic diagnostic apparatus according to claim 10, further comprising a switch configured to turn ON/OFF feedback control of the feedback parameter determining unit (38).

12. A method for estimating a viscosity parameter of an ultrasonic diagnostic apparatus, the method comprising:
applying an acoustic radiation pressure to an interior of an examinee for displacement;
transmitting and receiving an ultrasonic wave at a plurality of positions of the examinee to detect a particle velocity of a shear wave generated in the interior of the examinee; and
estimating a viscosity parameter from a temporal extent of a waveform of the detected particle velocity, **characterized in that**, based on the viscosity parameter estimated from a temporal extent of a waveform of the particle velocity obtained in a first measurement, a feedback parameter determining unit (38) adjusts at least one of a transmission condition and a region of interest in a second measurement, which is a subsequent measurement.

13. The method for estimating a viscosity parameter according to claim 12, wherein a temporal extent of a waveform of the particle velocity is calculated from two time instants, a time instance at which the particle velocity takes a positive value and a time instance at which the particle velocity takes a negative value, or a time instant at which the particle velocity takes a zero cross value, or an integral value of the particle velocity before and after a time instant at which the zero cross value is taken.

## Patentansprüche

1. Ultraschalldiagnosevorrichtung, die Folgendes umfasst:
eine Verschiebungserzeugungseinheit (10), die konfiguriert ist, einen Schallstrahlungsdruck auf ein Inneres einer zu untersuchenden Person anzuwenden, um eine Verschiebung zu erzeugen;
eine Verschiebungsdetektionseinheit (30), die konfiguriert ist, eine Ultraschallwelle an mehrere Positionen der zu untersuchenden Person zu übertragen und an mehreren Positionen der zu untersuchenden Person zu empfangen, um eine Teilchengeschwindigkeit einer Transversalwelle, die in dem Inneren der zu untersuchenden Person erzeugt worden ist, zu detektieren; und
eine Viskoelastizität-Analysiereinheit (34), die konfiguriert ist, aus der Teilchengeschwindigkeit einen Viskositätsparameter zu schätzen,
wobei die Viskoelastizität-Analysiereinheit (34) den Viskositätsparameter aus einer zeitlichen Ausdehnung der Wellenform der Teilchengeschwindigkeit schätzt, **dadurch gekennzeichnet, dass** die Vorrichtung ferner eine Rückkopplungsparameter-Bestimmungseinheit (38) umfasst,
wobei die Rückkopplungsparameter-Bestimmungseinheit (38) basierend auf dem Viskositätsparameter, der aus einer zeitlichen Ausdehnung der Wellenform der Teilchengeschwindigkeit geschätzt wird, die in einer ersten Messung erhalten wird, eine Transmissionsbedingung und/ oder einen interessierenden Bereich in einer zweiten Messung, die eine nachfolgende Messung darstellt, einstellt.

2. Ultraschalldiagnosevorrichtung nach Anspruch 1, wobei die Viskoelastizität-Analysiereinheit (34) eine zeitliche Ausdehnung einer Wellenform der Teilchengeschwindigkeit aus zwei Zeitpunkten, einem Zeitpunkt, an dem die Teilchengeschwindigkeit einen positiven Wert annimmt, und einem Zeitpunkt, an dem die Teilchengeschwindigkeit einen negativen Wert annimmt, berechnet.

3. Ultraschalldiagnosevorrichtung nach mindestens einem der Ansprüche 1 bis 2, wobei die Viskoelastizität-Analysiereinheit (34) eine zeitliche Ausdehnung einer Wellenform der Teilchengeschwindigkeit aus einem Integralwert der Teilchengeschwindigkeit zu einem Zeitpunkt, zu dem die Teilchengeschwindigkeit einen Nulldurchgangswert annimmt, oder vor und nach einem Zeitpunkt, zu dem der Nulldurchgangswert angenommen wird, berechnet.

4. Ultraschalldiagnosevorrichtung nach mindestens einem der Ansprüche 1 bis 3, wobei die Viskoelastizität-Analysiereinheit (34) den Viskositätsparameter basierend auf einer zeitlichen Ausdehnung einer Wellenform der Teilchengeschwindigkeit, die an den mehreren Positionen berechnet wird, durch Anpassung schätzt.

5. Ultraschalldiagnosevorrichtung nach Anspruch 4, wobei die Viskoelastizität-Analysiereinheit (34) das Anpassen unter Verwendung einer Exponentialkurve durchführt.

6. Ultraschalldiagnosevorrichtung nach mindestens einem der Ansprüche 1 bis 5, wobei die Viskoelastizität-Analysiereinheit (34) eine Mittenfrequenz der Teilchengeschwindigkeit berechnet.

7. Ultraschalldiagnosevorrichtung nach mindestens einem der Ansprüche 1 bis 6, wobei die Teilchengeschwindigkeit durch eine Korrelationsoperation zwischen einem einzelnen Referenzsignal und mehreren Signalen, die an der Verschiebungsdetektionseinheit (30) an unterschiedlichen Zeitpunkten empfangen werden, berechnet wird.

8. Ultraschalldiagnosevorrichtung nach mindestens einem der Ansprüche 1 bis 7, wobei die Teilchengeschwindigkeit durch eine Korrelationsoperation zwischen mehreren Signalen, die an der Verschiebungsdetektionseinheit (30) an unterschiedlichen Zeitpunkten empfangen werden, berechnet wird.

9. Ultraschalldiagnosevorrichtung nach Anspruch 8, wobei die Rückkopplungsparameter-Bestimmungseinheit (38) eine Größe des interessierenden Bereichs verändert, so dass er entsprechend einer Messposition eingestellt wird.

10. Ultraschalldiagnosevorrichtung nach Anspruch 9, wobei eine Form des interessierenden Bereichs einem Trapez entspricht.

11. Ultraschalldiagnosevorrichtung nach Anspruch 10, die ferner einen Schalter umfasst, der konfiguriert ist, die Rückkopplungssteuerung der Rückkopplungsparameter-Bestimmungseinheit (38) ein- bzw. auszuschalten.

12. Verfahren zum Bestimmen eines Viskositätsparameters einer Ultraschalldiagnosevorrichtung, wobei das Verfahren Folgendes umfasst:
Anwenden eines Schallstrahlungsdrucks auf ein Inneres einer zu untersuchenden Person, um eine Verschiebung zu bewirken;
Übertragen einer Ultraschallwelle an mehrere Positionen und Empfangen einer Ultraschallwelle an mehreren Positionen der zu untersuchenden Person, um eine Teilchengeschwindigkeit einer Transversalwelle, die in dem Inneren der zu untersuchenden Person erzeugt wird, zu detektieren; und
Schätzen eines Viskositätsparameters aus einer zeitlichen Ausdehnung einer Wellenform der detektierten Teilchengeschwindigkeit, **dadurch gekennzeichnet, dass**
die Rückkopplungsparameter-Bestimmungseinheit (38) basierend auf dem Viskositätsparameter, der aus einer zeitlichen Ausdehnung einer Wellenform der Teilchengeschwindigkeit, die in einer ersten Messung erhalten wird, in einer zweiten Messung, die eine nachfolgende Messung ist, eine Transmissionsbedingung und/oder einen interessierenden Bereich einstellt.

13. Verfahren zum Schätzen eines Viskositätsparameters nach Anspruch 12, wobei eine zeitliche Ausdehnung einer Wellenform der Teilchengeschwindigkeit aus zwei Zeitpunkten berechnet wird, einem Zeitpunkt, zu dem die Teilchengeschwindigkeit einen positiven Wert annimmt, und einem Zeitpunkt, zu dem die Teilchengeschwindigkeit einen negativen Wert annimmt, oder einem Zeitpunkt, zu dem die Teilchengeschwindigkeit einen Nulldurchgangswert annimmt, oder einen Integralwert der Teilchengeschwindigkeit vor und nach einem Zeitpunkt, zu dem der Nulldurchgangswert angenommen wird.

## Revendications

1. Appareil de diagnostic par ultrasons comprenant :
une unité de génération de déplacement (10) configurée pour appliquer une pression de rayonnement acoustique un intérieur d'un échantillon examiné pour provoquer un déplacement;
une unité de détection de déplacement (30) configurée pour transmettre et recevoir une onde ultrasonore à une pluralité de positions de l'échantillon examiné pour détecter une vitesse des particules d'une onde de cisaillement générée dans l'intérieur de l'échantillon examiné; et
une unité d'analyse de viscoélasticité (34) configurée pour estimer un paramètre de viscosité à partir de la vitesse des particules,
dans lequel l'unité d'analyse de viscoélasticité (34) estime le paramètre de viscosité à partir d'une extension temporelle d'une forme d'onde de la vitesse des particules,
**caractérisé en ce qu'**il comprend en outre une unité de détermination de paramètre de rétroaction (38),
dans lequel, en se basant sur le paramètre de viscosité estimé à partir d'une extension temporelle d'une forme d'onde de la vitesse des particules obtenues dans une première mesure, l'unité de détermination de paramètre de rétroaction (38) ajuste au moins un paramètre parmi une condition de transmission et une région présentant un intérêt dans une seconde mesure, qui est une mesure ultérieure.

2. Appareil de diagnostic par ultrasons selon la revendication 1, dans lequel l'unité d'analyse de viscoélasticité (34) calcule une extension temporelle d'une forme d'onde de la vitesse des particules à partir de deux instants dans le temps, d'une instance temporelle à laquelle la vitesse des particules adopte une valeur positive et une instance temporelle à laquelle la vitesse des particules adopte une valeur négative.

3. Appareil de diagnostic par ultrasons selon l'une au moins des revendications 1 et 2, dans lequel l'unité d'analyse de viscoélasticité (34) calcule une extension temporelle d'une forme d'onde de la vitesse des particules à partir d'une valeur intégrale de la vitesse des particules à un instant dans le temps auquel la vitesse des particules adopte une valeur qui croise zéro ou avant et après un instant dans le temps auquel la valeur qui croise zéro est adoptée.

4. Appareil de diagnostic par ultrasons selon l'une au moins des revendications 1 à 3, dans lequel l'unité d'analyse de viscoélasticité (34) estime le paramètre de viscosité par un ajustement basé sur une extension temporelle d'une forme d'onde de la vitesse des particules calculées à la pluralité de positions.

5. Appareil de diagnostic par ultrasons selon la revendication 4, dans lequel l'unité d'analyse de viscoélasticité (34) exécute l'ajustement en utilisant une courbe exponentielle.

6. Appareil de diagnostic par ultrasons selon l'une au moins des revendications 1 à 5, dans lequel l'unité d'analyse de viscoélasticité (34) calcule une fréquence centrale de la vitesse des particules.

7. Appareil de diagnostic par ultrasons selon l'une au moins des revendications 1 à 6, dans lequel la vitesse des particules est calculée par une opération de corrélation entre un unique signal de référence et une pluralité de signaux reçus à l'unité de détection de déplacement (30) à différents instants dans le temps.

8. Appareil de diagnostic par ultrasons selon l'une au moins des revendications 1 à 7, dans lequel la vitesse des particules est calculée par une opération de corrélation entre une pluralité de signaux reçus à l'unité de détection de déplacement (30) à différents instants dans le temps.

9. Appareil de diagnostic par ultrasons selon la revendication 8, dans lequel l'unité de détermination de paramètre de rétroaction (38) change une taille de la région présentant un intérêt à ajuster en correspondance d'une position de mesure.

10. Appareil de diagnostic par ultrasons selon la revendication 9, dans lequel une forme de la région présentant un intérêt est une forme trapézoïdale.

11. Appareil de diagnostic par ultrasons selon la revendication 10, comprenant en outre un commutateur configuré pour commuter en marche/arrêt la commande de rétroaction de l'unité de détermination de paramètre de rétroaction (38).

12. Procédé pour estimer un paramètre de viscosité d'un appareil de diagnostic par ultrasons, le procédé comprenant les étapes consistant à :
appliquer une pression de rayonnement acoustique à un intérieur d'un échantillon examiné en vue d'un déplacement ;
transmettre et recevoir une onde ultrasonore à une pluralité de positions de l'échantillon examiné pour détecter une vitesse des particules d'une onde de cisaillement générée dans l'intérieur de l'échantillon examiné; et
estimer un paramètre de viscosité à partir d'une extension temporelle d'une forme d'onde de la vitesse des particules détectée,
**caractérisé en ce que**
en se basant sur le paramètre de viscosité estimé à partir d'une extension temporelle d'une forme d'onde de la vitesse des particules obtenue dans une première mesure, une unité de détermination de paramètre de rétroaction (38) ajuste au moins un paramètre parmi une condition de transmission et une région présentant un intérêt dans une seconde mesure, qui est une mesure ultérieure.

13. Procédé pour estimer un paramètre de viscosité selon la revendication 12, dans lequel une extension temporelle d'une forme d'onde de la vitesse des particules est calculée à partir de deux instants dans le temps, une instance temporelle à laquelle la vitesse des particules adopte une valeur positive et une instance temporelle à laquelle la vitesse des particules adopte une valeur négative, ou un instant dans le temps auquel la vitesse des particules adopte une valeur qui croise zéro, ou une valeur intégrale de la vitesse des particules avant et après un instant dans le temps auquel la valeur qui croise zéro est adoptée.
